# EUROPÄISCHE PATENTSCHRIFT

(11) **EP 1 073 487 B1**
(45) Veröffentlichungstag und Bekanntmachung des Hinweises auf die Patenterteilung: **20.07.2005**
(21) Anmeldenummer: 99915530.2
(22) Anmeldetag: 20.02.1999
(51) Int. Cl.: A61M 1/02

(54) **VERFAHREN ZUR HERSTELLUNG EINES THROMBOZYTENPRÄPARATES**
METHOD FOR PRODUCING A THROMBOCYTE PREPARATION
PROCEDE DE PRODUCTION D'UNE PREPARATION PLAQUETTAIRE

(30) Priorität: 18.04.1998 DE 19817328
(43) Veröffentlichungstag der Anmeldung: 07.02.2001
(73) Patentinhaber: Eberhard-Karls-Universität Tübingen Universitätsklinikum, 72076 Tübingen (DE)
(72) Erfinder: HÖRNLEIN, Rainer, Frank, D-72076 Tübingen (DE)
(74) Vertreter: Otten, Hajo, Dr.-Ing.
(86) Internationale Anmeldenummer: PCT/EP1999/001119
(87) Internationale Veröffentlichungsnummer: WO 1999/053975

(56) Entgegenhaltungen:
- WO-A-84/00492
- WO-A-91/04088
- WO-A-97/07836

## Beschreibung

Die vorliegende Erfindung betrifft ein Verfahren zur Herstellung eines leukozyten- und erythrozytenarmen Thrombozytenpräparates, mit den Schritten:
- Herstellen eines Thrombozytenkonzentrats, das Leukozyten und einen restlichen Anteil an Erythrozyten enthält,
- Zentrifugieren des Thrombozytenkonzentrats,
- Filtern des Thrombozytenkonzentrats durch einen Leukozytendepletionsfilter, wobei mit dem Zentrifugationsüberstand begonnen wird, und
- Abtrennung des Erythrozytenanteils.

Ein derartiges Verfahren ist bekannt aus der Veröffentlichung "Christensen und Dickmeiss: In vitro Evaluation of a New Filter for Leucocyte Depletion of Platelet Concentrate during Component Preparation", Vox Sang 1994; 67; 267-271.

Ferner beschreibt die WO-A-97/07836 ein Verfahren zur Separation von Thrombozytenkonzentrat aus Buffy Coat, bei dem während der mechanischen Abtrennung des Thrombozytenkonzentrats und der Überführung der thrombozytenreichen Fraktion in einen Konzentratbeutel optisch der Gehalt an Erythrozyten im Schlauchsystem zwischen Buffy Coat Beutel und dem Konzentratbeutel überwacht wird.

Derartige Thrombozytenpräparate sind aus Frischblut gewonnene, von thrombozytenreichem Plasma hergestellte Blutkonserven, bei denen 50 ml ca. 10¹¹ Thrombozyten enthalten. Die Präparate werden durch Transfusion einem Patienten zur Aufrechterhaltung der Hämostase z.B. bei Störungen der Thrombozytopoese beispielsweise bei Leukämien oder Knochenmarkkarzinose verabreicht. Bei intensiver Zytostatikatherapie werden die Präparate auch zur Blutungsprophylaxe angewendet.

Die Haltbarkeit der Thrombozytenpräparate liegt bei wenigen Tagen, so daß diese für den Klinikalltag insbesondere von Transplantations- und Krebszentren wichtige Blutkonserve nicht in großem Umfang auf Vorrat gehalten sondern ständig frisch hergestellt werden muß. Die Herstellung geht dabei nicht von frischem Vollblut sondern neuerdings von sogenannten Buffy Coats aus, also der nach dem Zentrifugieren von Vollblut entstehenden Schicht aus Leukozyten und Thrombozyten zwischen dem Plasmaüberstand und den sedimentierten Erythrozyten. Nach der Zentrifugation werden z.B. in einer Blutbeutelpresse das Plasma nach oben und die Erythrozyten nach unten abgepreßt, bis in dem so abgepreßten Blutbeutel nur noch der Buffy Coat übrig bleibt, in dem Thrombozyten, Leukozyten sowie ein Restanteil von Erythrozyten enthalten sind.

Zur Weiterverarbeitung werden dann mehrere Blutbeutel mit Buffy Coat hintereinander geschaltet aufgehängt, wobei in den oberen Blutbeutel Plasma oder eine additive Lösung zum Durchspülen der untereinander hängenden Blutbeutel eingegeben wird. Aus den oberen Blutbeuteln wird auf diese Weise der Buffy Coat herausgespült, so daß sich im unteren Blutbeutel ein Thrombozytenkonzentrat bildet, das Leukozyten sowie einen restlichen Anteil an Erythrozyten enthält.

Bevor das Erythrozytenkonzentrat allgemein eingesetzt werden kann, muß zum einen die Erythrozytenkontamination stark verringert werden, um Transfusionszwischenfälle z.B. durch Blutgruppenantikörper im Empfängerblut zu verhindern.

Aber auch die Zahl der Leukozyten muß stark verringert werden, um Transfusionszwischenfälle z.B. durch Alloantikörper des Empfängers gegen die Leukozyten zu vermeiden. Auch um Virusübertragungen etc. zu vermeiden, muß bei dem Thrombozytenpräparat eine Leukozytendepletion durchgeführt werden.

Bei dem aus der eingangs genannten Veröffentlichung bekannten Verfahren wird das aus den gepoolten Buffy Coats gewonnene Thrombozytenkonzentrat ausgehend vom Zentrifugationsüberstand mittels eines sogenannten AutoStop™ BC-Filter gefiltert, der eine Leukozytendepletion durchführt und die Erythrozyten von den Thrombozyten abtrennt. Die Leukozyten werden dabei in dem Filter festgehalten, während die Erythrozyten aufgrund ihrer Oberflächenladung abgestoßen werden und den Filter nicht passieren können.

Der erwähnte AutoStop™ BC-Filter ist nur von der Firma Pall Biomedical Ltd., Portsmouth, UK erhältlich, vergleichbare Filter sind nach Kenntnis der Anmelderin nicht auf dem Markt.

Bei dem bekannten AutoStop™ BC-Filter ist zum einen sein hoher Preis von Nachteil, wobei der Erfinder der vorliegenden Anmeldung ferner festgestellt hat, daß die Leukozytendepletion und Erythrozytenabtrennung im Alltagseinsatz häufig nicht die in der oben genannten Veröffentlichung angegebenen Werte erreicht, was aus den oben erwähnten Gründen medizinisch von Nachteil ist.

Vor diesem Hintergrund ist es Aufgabe der vorliegenden Erfindung, das bekannte Verfahren derart weiterzubilden, daß das Thrombozytenpräparat preiswerter hergestellt werden kann und eine bessere Leukozytendepletion sowie Erthrozytenabtrennung erreicht wird.

Erfindungsgemäß wird diese Aufgabe bei dem eingangs erwähnten Verfahren dadurch gelöst, daß das Thrombozytenkonzentrat in einem Blutbeutel gelagert wird, dessen Thrombozytenschlauch mit dem Leukozytendepletionsfilter verbunden ist, und beim Filtern der Blutbeutel einem Preßvorgang in einer Blutbeutelpresse unterzogen wird, daß ferner ein Erythrozytendetektor den Erythrozytenanteil in dem Thrombozytenschlauch überwacht, und daß eine Ablaufsteuerung der Blutbeutelpresse den Erythrozytendetektor abfragt und bei Erreichen eines bestimmten Erythrozytenanteils den Preßvorgang abstoppt.

Die der Erfindung zugrunde liegende Aufgabe wird auf diese Weise vollkommen gelöst.

Der Erfinder der vorliegenden Anmeldung hat nämlich erkannt, daß durch die Überwachung des Erythrozytenanteils vor dem Leukozytendepletionsfilter und durch sozusagen rechtzeitige Beendigung des gesamten Filtervorgangs eine deutlich bessere Erythrozytenabtrennung erreicht wird als bei dem bekannten Verfahren. Als Leukozytendepletionsfilter kann ein konventionelles Leukozytendepletionsfilter verwendet werden, das bezüglich der Luekozytendepletion optimiert ist. Auf diese Weise ergibt sich verglichen mit dem bekannten Verfahren eine deutlich geringere Restkonzentration an Leukozyten und Erythrozyten in dem Thrombozytenpräparat.

Die Filterung kann dabei z.B. durch Schwerkraft erfolgen, wobei ein Detektor optisch das Eintreffen der ersten Erythrozyten vor dem Filter anzeigt. Bedienungspersonal kann dann z.B. über eine Schlauchklemme den Trombozytenschlauch vor dem Leukozytendepletionsfilter schließen. Obwohl auf diese Weise ggf. nicht das gesamte Trombozytenkonzentrat gefiltert wird, ergibt sich nach Erkenntnis des Erfinders doch eine sehr hohe Ausbeute an Trombozyten, die ca. 10 % über der Ausbeute bei dem bekannten Verfahren liegt.

Auf diese Weise bietet das Verfahren eine größere Sicherheit bei der Leukozytendepletion sowie der Erythrozytenabtrennung. Ferner ist zu erwähnen, daß konventionelle Leukozytendepletionsfilter nur ungefähr ein Drittel so teuer sind wie der eingangs erwähnte AutoStop**™** BC-Filter, da die Autostop-Funktion für die Erythrozyten nicht erforderlich ist. Dies bedeutet jedoch, daß das neue Verfahren deutlich preiswerter ist, da für jedes herzustellende Thrombozytenpräparat ein neuer Leukozytendepletionsfilter eingesetzt werden muß.

Derartige Leukozytendepletionsfilter sind z.B. über die Fresenius AG, 61343 Bad Homburg unter der Bezeichnung BioP plus BBS PF erhältlich.

Der Leukozytendepletionsfilter von Fresenius wird in einem System mit Blutbeutel sowie Bypass- oder AutoVenting-Anordnung vertrieben, bei dem zusätzlich zu dem Leukozytendepletionsfilter und dem diesen mit dem Blutbeutel verbindenden Schlauch noch weitere Schläuche und sterile Belüftungsmöglichkeiten vorgesehen sind.

Das Thrombozytenkonzentrat wird dabei in einem Blutbeutel gelagert, dessen Thrombozytenschlauch mit dem Leukozytendepletionsfilter verbunden wird, und beim Filtern der Blutbeutel einem Preßvorgang in einer Blutbeutelpresse unterzogen wird.

So kann insgesamt ein sehr einfaches System verwendet werden; an den Thrombozytenschlauch eines Blutbeutels muß lediglich der Leukozytendepletionsfilter angeschlossen werden, an den der Sammelbeutel für das Thrombozytenpräparat angeschlossen ist. Durch den Preßvorgang in der Blutbeutelpresse wird das Thrombozytenkonzentrat dann durch den Leukozytendepletionsfilter gepreßt, was insgesamt einen sehr schnellen Preßvorgang ermöglicht. Bei dem eingangs erwähnten BioP plus BBS PF sind damit lediglich noch der Filter sowie der Sammelbeutel erforderlich, auf die zusätzlichen Komponenten für das Bypass- oder AutoVenting-System kann verzichtet werden, was die Kosten pro Thrombozytenpräparat weiter senkt.

Dabei überwacht ein Erythrozytendetektor den Erythrozytenanteil in dem Thrombozytenschlauch, wobei eine Ablaufsteuerung der Blutbeutelpresse den Erythrozytendetektor abfragt und bei Erreichen eines bestimmten Erythrozytenanteils den Preßvorgang abstoppt.

In einer Weiterbildung ist bevorzugt, wenn die Ablaufsteuerung nach dem Abstoppen des Preßvorgangs eine Schweißeinheit aktiviert, die den Thrombozytenschlauch vor dem Leukozytendepletionsfilter abklemmt und verschweißt.

Diese Maßnahmen lassen sich vorteilhaft auf einer Blutbeutelpresse mit Erythrozytendetektor durchführen, so daß eine konventionelle Blutbeutelpresse verwendet werden kann, um den Filtervorgang zu treiben und bei Erreichen einer bestimmten Erythrozytenkonzentration abzupressen. Durch das Verschweißen des Thrombozytenschlauchs wird auch eine Kontamination durch Diffusion von Erythrozyten verhindert, so daß hier insgesamt durch das zielgenaue Beenden des Preßvorgangs und Verschweißen des Thrombozytenschlauchs eine sehr gute Erythrozytenabtrennung erreicht wird.

Dabei ist es noch bevorzugt, wenn nach dem Abklemmen des Erythrozytenschlauchs der Leukozytendepletionsfilter steril belüftet wird.

Hier ist von Vorteil, daß auch noch das beim Abstoppen des Preßvorgangs in dem Leukozytendepletionsfilter sowie dem Verbindungsschlauch zu dem Lagerbeutel befindliche Thrombozytenpräparat gewonnen werden kann, wodurch die Ausbeute an Thrombozyten erhöht wird.

Die Erfindung betrifft ferner die Verwendung einer Blutbeutelpresse, die vorzugsweise einen Erythrozytendetektor aufweist, zur Herstellung eines leukozyten- und erythrozytenarmen Thrombozytenpräparats aus einem Thrombozytenkonzentrat, das Leukozyten und einen restlichen Anteil an Erythrozyten enthält, wobei auf dieser Blutbeutelpresse das neue Verfahren durchgeführt wird.

Die Erfindung betrifft ferner die Verwendung eines Leukozytendepletionsfilters, der vorzugsweise eine sterile Belüftungsmöglichkeit vorsieht, zur Herstellung eines leukozyten- und erythrozytenarmen Thrombozytenpräparats aus einem Thrombozytenkonzentrat, das Leukozyten und einen restlichen Anteil an Erythrozyten enthält, wobei der Leukozytendepletionsfilter bei dem neuen Verfahren verwendet wird.

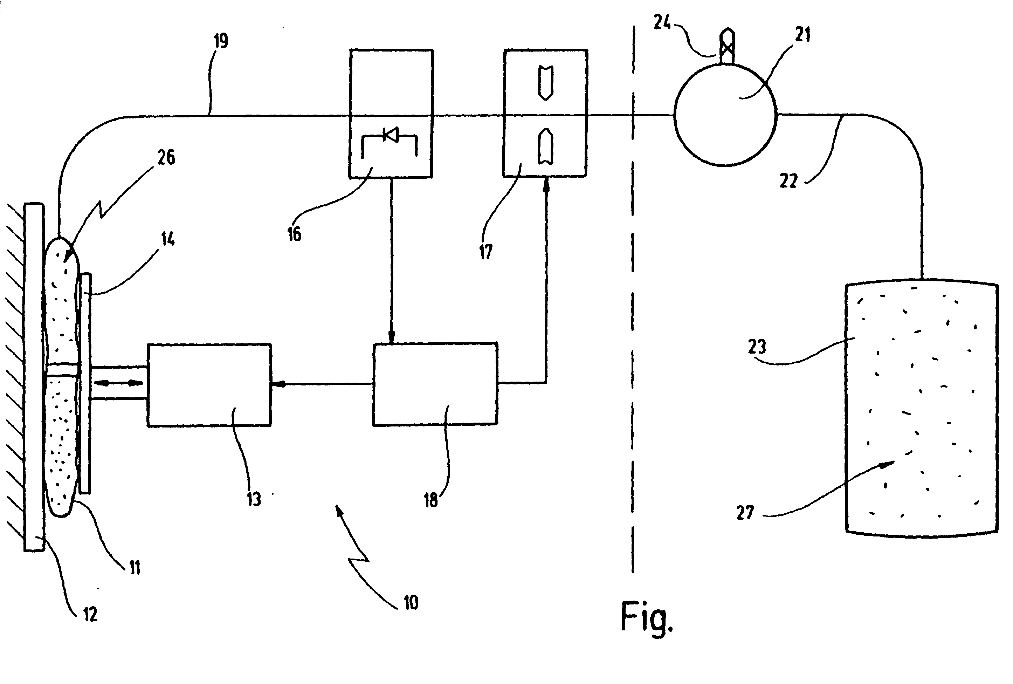

Weitere Vorteile ergeben sich aus der Beschreibung und der beigefügten Zeichnung.

Ein Ausführungsbeispiel der Erfindung ist in der Zeichnung dargestellt und wird in der nachfolgenden Beschreibung näher erläutert.

Die einzige Figur zeigt den Einsatz einer Blutbeutelpresse mit Erythrozytendetektor zur Herstellung eines Thrombozytenpräparats unter Verwendung eines Leukozytendepletionsfilters.

In der einzigen Figur ist links von der gestrichelten Linie sehr schematisch eine übliche Blutbeutelpresse 10 gezeigt, die einen bei 11 angedeuteten Blutbeutel zwischen einer stehenden Platte 12 und einer durch eine Kolben-Zylinder-Einheit 13 angetriebene bewegliche Platte 14 abpreßt.

Die Blutbeutelpresse 10 umfaßt ferner einen Erythrozytendetektor 16, der auch als Rot- oder Hb-Detektor bezeichnet wird. Ferner ist auch eine Schweißeinheit 17 vorgesehen, die in der Figur lediglich schematisch angedeutet ist.

Die Blutbeutelpresse 10 umfaßt ferner eine Steuereinheit 18, die mit der Kolben-Zylinder-Einheit 13, dem Erythrozytendektektor 16 sowie der Schweißeinheit 17 verbunden ist.

Von dem Blutbeutel 11 geht ein Thrombozytenschlauch 19 aus, der zunächst durch den Erythrozytendetektor 16 und dann durch die Schweißeinheit 17 verläuft, bevor er dann zu einem Leukozytendepletionsfilter 21 gelangt. Von dem Leukozytendepletionsfilter 21 geht ein weiterer Schlauch aus, durch den das Filtrat in einen Sammelbeutel 23 fließt. An dem Leukozytendepletionsfilter 21 ist bei 24 noch ein Ventil zur sterilen Belüftung angedeutet.

Durch die insoweit beschriebene Anordnung wird jetzt durch die folgenden Schritte aus einem in dem Blutbeutel 11 befindlichen Thrombozytenkonzentrat 26 ein leukozyten- und erythrozytenarmes Thrombozytenpräparat 27 hergestellt, das in dem Sammelbeutel 23 angedeutet ist.

Das Thrombozytenkonzentrat 26 ist auf die eingangs erwähnte Weise durch Poolen von z.B. vier Buffy Coats und Spülen mit additiver Lösung hergestellt worden und enthält sowohl Leukozyten als auch einen restlichen Anteil an Erythrozyten. Vor dem Einspannen des Blutbeutels 11 in die Blutbeutelpresse 10 wurde der Blutbeutel 11 noch zentrifugiert, so daß sich ein plättchenreicher Überstand, eine Grenzschicht sowie sedimentierte Erythrozyten in der üblichen Weise voneinander getrennt haben.

Die Steuereinheit 18 betätigt jetzt die Kolben-Zylinder-Einheit 13 derart, daß diese die bewegliche Platte 14 auf die stehende Platte 12 zu bewegt und dabei den Blutbeutel 11 abpreßt. Auf diese Weise gelangt zunächst der plättchenreiche Überstand durch den Thrombozytenschlauch 18 an dem Erythrozytendetektor 16 sowie der Schweißeinheit 17 vorbei zu dem Leukozytendepletionsfilter 21 und von dort in den Sammelbeutel 23. Die in dem Überstand vorhandenen Leukozyten werden in dem Leukozytendepletionsfilter 21 zurückgehalten, wobei in dem Überstand auch nur sehr wenige Erythrozyten vorhanden sind, so daß in dem Thrombozytenpräparat 27 nur geringe Konzentrationen an Leukozyten und Erythrozyten zu finden sind.

Nach dem Überstand gelangt die Grenzschicht in den Thrombozytenschlauch 19, wobei die in der Grenzschicht enthaltenen Leukozyten ebenfalls durch den Leukozytendepletionsfilter 21 zurückgehalten werden.

Beim weiteren Abpressen gelangen dann auch Erythrozyten in den Thrombozytenschlauch 19, was der Erythrozytendetektor 16 erkennt und an die Steuereinheit 18 meldet. Sobald in der an dem Erythrozytendetektor 16 vorbeifließenden Lösung ein bestimmter Erythrozytenanteil enthalten ist, stoppt die Steuereinheit 18 den Abpreßvorgang durch einen entsprechenden Befehl an die Kolben-Zylinder-Einheit 13 und betätigt daraufhin die Schweißeinheit 17 derart, daß der Thrombozytenschlauch 19 vor dem Leukozytendepletionsfilter 21 abgetrennt und verschweißt wird.

Durch die Einstellung der Empfindlichkeitsschwelle des Erythrozytendetektors 16 kann auf diese Weise die Erythrozytenkontamination in dem Sammelbeutel 23 sehr genau eingestellt werden.

Nach dem Abtrennen des Thrombozytenschlauchs 19 wird das Ventil 24 geöffnet, so daß sich das bereits in dem Leukozytendepletionsfilter 21 bzw. in dem Schlauch 22 befindliche, gefilterte Leukozytenpräparat in den Sammelbeutel 23 entleeren kann.

Erste Versuche mit dem neuen Verfahren haben ergeben, daß aus vier Buffy Coats bereits eine Thrombozytenausbeute von 3 x 10¹¹ erreicht werden kann, was standardmäßig nur mit fünf Buffy Coats möglich ist. Ferner liegt nach Erkenntnissen des Erfinders die Erythrozytenkontamination in derselben Größenordnung wie bei dem eingangs erwähnten Verfahren, während die Leukozytenkonzentration nahezu um einen Faktor 10 geringer ist als bei dem bekannten Verfahren.

## Patentansprüche

1. Verfahren zur Herstellung eines leukozyten- und erythrozytenarmen Thrombozytenpräparats (27), mit den Schritten:
- Herstellen eines Thrombozytenkonzentrats (26), das Leukozyten und einen restlichen Anteil an Erythrozyten enthält,
- Zentrifugieren des Thrombozytenkonzentrats (26),
- Filtern des Thrombozytenkonzentrats (26) durch einen Leukozytendepletionsfilter (21), wobei mit dem Zentrifugationsüberstand begonnen wird, und
- Abtrennung des Erythrozytenanteils,
**dadurch gekennzeichnet, daß** das Thrombozytenkonzentrat (26) in einem Blutbeutel (11) gelagert wird, dessen Thrombozytenschlauch (19) mit dem Leukozytendepletionsfilter (21) verbunden ist, und beim Filtern der Blutbeutel (11) einem Preßvorgang in einer Blutbeutelpresse (10) unterzogen wird, daß ferner ein Erythrozytendetektor (16) den Erythrozytenanteil in dem Thrombozytenschlauch (19) überwacht, und daß eine Ablaufsteuerung (18) der Blutbeutelpresse (10) den Erythrozytendetektor (16) abfragt und bei Erreichen eines bestimmten Erythrozytenanteils den Preßvorgang abstoppt.

2. Verfahren nach Anspruch 1, **dadurch gekennzeichnet, daß** die Ablaufsteuerung (18) nach dem Abstoppen des Preßvorgangs eine Schweißeinheit (17) aktiviert, die den Thrombozytenschlauch (19) vor dem Leukozytendepletionsfilter (21) abklemmt und verschweißt.

3. Verfahren nach Anspruch 2, **dadurch gekennzeichnet, daß** nach dem Abklemmen des Thrombozytenschlauchs (19) der Leukozytendepletionsfilter (21) steril belüftet wird.

4. Verwendung einer Blutbeutelpresse (10), die vorzugsweise einen Erythrozytendetektor (16) aufweist, zur Herstellung eines leukozyten- und erythrozytenarmen Thrombozytenpräparates (27) aus einem Thrombozytenkonzentrat (26), das Leukozyten und einen restlichen Anteil an Erythrozyten enthält, bei einem Verfahren nach einem der Ansprüche 1 bis 3.

5. Verwendung eines Leukozytendepletionsfilters (21), der vorzugsweise eine sterile Belüftungsmöglichkeit (24) aufweist, zur Herstellung eines leukozyten- und erythrozytenarmen Thrombozytenpräparats (27) aus einem Thrombozytenkonzentrat (26), das Leukozyten und einen restlichen Anteil an Erythrozyten enthält, bei einem Verfahren nach einem der Ansprüche 1 bis 3.

## Claims

1. Method for producing a thrombocyte preparation (27) depleted in leucocytes and erythrocytes, comprising the steps of:
- producing a thrombocyte concentrate (26) containing leucocytes and a remainder of erythrocytes,
- centrifuging the thrombocyte concentrate (26),
- filtering the thrombocyte concentrate through a leucocyte depletion filter (21) starting with the top fraction of the centrifugated concentrate, and
- separating the erythrocyte component,
**characterized in that** the thrombocyte concentrate (26) is stored in a blood bag (11), whose thrombocyte tube (19) is connected to the leucocyte depletion filter (21) and wherein the blood bag (11) is subjected to pressing in a blood bag press (10) during filtration, and further, that an erythrocyte detector (16) monitors the erythrocyte concentration in the thrombocyte tube (19), and that a process controller (18) of the blood bag press (10) accesses the erythrocyte detector (16) and stops the pressing procedure when a certain erythrocyte concentration is reached.

2. Method of claim 1, wherein the process controller (18) activates a welding unit (17) after stopping the pressing process, which clamps off and welds the thrombocyte tube (19) at a location before the leucocyte depletion filter (21).

3. Method of claim 2, **characterized in that** the leucocyte depletion filter (21) is ventilated under sterile conditions after clamping off the thrombocyte tube (19).

4. Use of a blood bag press (10), preferably comprising an erythrocyte detector (16) for producing a leucocyte and erythrocyte depleted thrombocyte preparation (27) from a thrombocyte concentrate (26), which contains leucocytes and a remainder of erythrocytes, in the method of one of the claims 1 to 3.

5. Use of a leucocyte depletion filter (21), preferably with the possibility of sterile ventilation (24), for producing a leucocyte and erythrocyte depleted thrombocyte preparation (27) from a thrombocyte concentrate (26), which contains leucocytes and a remainder of erythrocytes, in the method of one of the claims 1 to 3.

## Revendications

1. Procédé de production d'une préparation plaquettaire appauvrie en leucocytes et en érythrocytes (27), comprenant les étapes consistant à :
- produire un concentré plaquettaire (26), qui comprend des leucocytes et une proportion résiduelle d'érythrocytes,
- centrifuger le concentré plaquettaire (26),
- filtrer le concentré plaquettaire (26) avec un filtre de déleucocytation (21), en commençant par le surnageant de centrifugation, et
- séparer la proportion d'érythrocytes,
**caractérisé en ce que** le concentré plaquettaire (26) est stocké dans une poche de sang (11), dont le tuyau de produit plaquettaire (19) est relié au filtre de déleucocytation (21), et est soumis, lors du filtrage des poches de sang (11), à un procédé de pression dans une presse à poches de sang (10), **en ce qu'**un détecteur d'érythrocytes (16) surveille en outre la proportion d'érythrocytes présente dans le tuyau de produit plaquettaire (19), et **en ce qu'**une commande de routine (18) de la presse à poches de sang (10) fait une requête auprès du détecteur d'érythrocytes (16) et arrête le procédé de pression quand une certaine proportion d'érythrocytes est atteinte.

2. Procédé selon la revendication 1, **caractérisé en ce que**, après l'arrêt du procédé de pression, la commande de routine (18) active une unité de scellage (17) qui sépare par pression et soude le tuyau de produit plaquettaire (19) avant le filtre de déleucocytation (21).

3. Procédé selon la revendication 2, **caractérisé en ce que**, après la séparation par pression du tuyau de produit plaquettaire (19), le filtre de déleucocytation (21) est soumis à une ventilation stérile.

4. Utilisation d'une presse à poches de sang (10), qui présente de préférence un détecteur d'érythrocytes (16), pour la production d'une préparation plaquettaire appauvrie en leucocytes et en érythrocytes (27) à partir d'un concentré plaquettaire (26), qui comprend des leucocytes et une proportion résiduelle d'érythrocytes, dans un procédé selon l'une des revendications 1 à 3.

5. Utilisation d'un filtre de déleucocytation (21), qui présente de préférence une possibilité de ventilation stérile (24), pour la production d'une préparation plaquettaire appauvrie en leucocytes et en érythrocytes (27) à partir d'un concentré plaquettaire (26), qui comprend des leucocytes et une proportion résiduelle d'érythrocytes, dans un procédé selon l'une des revendications 1 à 3.
